# EUROPEAN PATENT APPLICATION

(11) **EP 0 877 031 A1**
(43) Date of publication of application: **11.11.1998**
(21) Application number: 97201342.9
(22) Date of filing: 06.05.1997
(51) Int. Cl.: C07K 14/495, A61K 38/18

(54) **TGF-Beta1 derived peptides mimicking the activity of transforming growth factor-Beta1**

(71) Applicant: INSTITUUT VOOR DIERHOUDERIJ EN DIERGEZONDHEID (ID-DLO), 8219 PH Lelystad (NL)
(72) Inventor: The designation of the inventor has not yet been filed
(74) Representative: Smulders, Theodorus A.H.J., Ir.

(57) **Abstract**

The present invention relates to a synthetic peptide derived from TGF-β and mimicking TGF-β activity, which peptide comprises at least 4 consecutive amino acids from one of the sequences CFSSTEKNCCVR, FSSTEKNCCVRQ, EKNCCVRQLYID, NCCVRQLYIDFR, CCVRQLYIDFRK, LYIDFRKDLGWK, FRKDLGWKWIHE, RKDLGWKWIHEP, HEPKGYHANFCL, TQYSKVLALYNQ, YSKVLALYNQHN, LYNQHNPGASAA, EQLSNMIVSCK, or LSNMIVRSCKCS, which peptide is between 4 and 18 amino acids in length; a pharmaceutical composition comprising said peptides as well as a pharmaceutical composition comprising TGF-β1 and at least one synthetic peptide derived from TGF-β and mimicking TGF-β activity, which peptide comprises at least 4 consecutive amino acids from one of the sequences KNCCVRQLYIDF, NCCVRQLYIDFR, CCVRQLYIDFRK, CVRQLYIDFRKD, VRQLYIDFRKDL, RQLYIDFRKDLG, HEPKGYHANFCL, EPKGYHANFCLG, TQYSKVLALYNQ, YSKVLALYNQH, KVLALYNQHNP, NQHNPGASAAPC, QHNPGASAAPCC, HNPGASAAPCCV, PKVEQLSNMIVR or LSNMIVRSCKCS, which peptide is between 4 and 18 amino acids in length, together with at least one suitable excipient for administration.

## Description

The present invention relates to novel peptides derived from the Transforming Growth Factor-β1(TGF-β1). These peptides have a TGF-β type activity; they are TGF-β agonists. Further, the invention relates to the combined use of TGF-β with peptides derived from TGF-β1. Finally, pharmaceutical compositions comprising the novel peptides are disclosed. Five homologues isoforms of TGF-β have been identified, which isoforms are referred toi as TGF-β1-TGF-β5. The vast majority of functional data concerning TGF's are on TGF-β1, which was discovered first.

It is known in the art that TGF-β has a number of interesting effects on human and animal cells.

Wahl et al. (Immunol. Today (1989) 258) and Sasaki et al. (Clin. Immunol. Immunopathol. (1992) 1) reported on indications that TGF-β has an important function in the regulation of immune response.

In addition, it has been shown by Kim et al. (J. Immunol. (1990), 3411 and J. Immunol. (1990), 3773) that TGF-β is involved in proliferative and anti-proliferative effects, chemotaxis, regulation of cytokine production and IgA isotype switching.

Because of the immunosuppressive properties of TGF-β1, it has been used in experimental models of autoimmune diseases, for example experimental allergic encephalomyelitis and collagen-induced arthritis, resulting in an improved clinical course. This is described by Racke et al. in J. Immunol. (1991), 3012, and by Kuruvilla et al. in Proc. Natl. Acad. Sci. (1991), 2918.

In addition, TGF-β, released by platelets, macrophages and other inflammatory cells, is involved in tissue repair. In this process, TGF-β plays a role in chemotaxis, proliferation and productional activity of monocytes, lymphocytes, fibroblasts and endothelial cells, resulting in angiogenesis and elaboration of connective tissue protein. Moreover, the destructive aspects of the inflammatory response, such as respiratory burst of macrophages, are suppressed by TGF-β. The important role of TGF-β in regulation of the inflammatory response has also been demonstrated since the disruption of mouse TGF-β1 gene results in a multifocal inflammatory disease, leading to organ failure and death. In this respect reference is made to Roberts et al. in Recent Progress in Hormone Research. (1990), 157; and in: Handbook of experimental Pharmacology. Editors: Sporn, M.B., Roberts, A.B., Heidelberg, Springer-Verlag (1990), 95:419, and to Shull et al. in Nature (1992), 693.

In embrionic development, TGF-β is thought to participate in the morphogenesis of many structures, as suggested by high expression of TGF-β in the mouse embryo in a unique pattern, both spatially and temporarily. See in this respect e.g. the afore mentioned publication of Roberts et al. Probably, TGF-β is particulary important in the differentiation of tissues of mesenchymal origin such as bone, muscle, blood vessels and blood cells, as well as in the differentiation of various epithelial tissues.

Furthermore, TGF-β is supposed to play a role in bone remodelling, regeneration of liver, muscles and nerves, hematopoiesis, steriodogenesis and carcinogenesis (Wahl et al. vide supra, and Fausto, Lab. Invest. (1991) 497). Besides this, a role for TGF-β has been proposed by e.g. Border et al. in: Ciba Found. Symp. (1991) clinical applications of TGF-β; 157:225, in the pathogenesis of a variety of connective tissue diseases such as keloids, cirrhosis, atherosclerosis, pulmonary fibrosis, scleroderma, rheumatoid arthritis and glomerulosclerosis (7,12).

One of the most fascinating aspects of the mode of action of TGF-β is that it has been indicated to have dissimilar or opposite effects on one, single, biologic process, depending on the cell type. Thus, therapeutical application of TGF-β indicates that opposite modes of action may be necessary in different diseases.

Because of these interesting effects of TGF-β, the present inventors aimed to find compounds which mimick or inhibit the activity of TGF-β for various clinical and veterinary applications. In particular, the inventors have tried to find synthetic peptides which can be administered in such a way that their systemic half-life time is sufficient to exert their activity before degradation occurs.

In accordance with the present invention it has been found that certain synthetic peptides derived from TGF-β1 mimick TGF-β1 activity. In addition, it has been found that certain synthetic peptides derived from TGF-β1 give a synergistic effect when these are used in combination with TGF-β1.

In a first aspect, the present invention relates to a synthetic peptide derived from TGF-β1 and mimicking TGF-β1 activity, which peptide comprises at least 4 consecutive amino acids from one of the sequences CFSSTEKNCCVR, FSSTEKNCCVRQ, EKNCCVRQLYID, NCCVRQLYIDFR, CCVRQLYIDFRK, LYIDFRKDLGWK, FRKDLGWKWIHE, RKDLGWKWIHEP, HEPKGYHANFCL, TQYSKVLALYNQ, YSKVLALYNQHN, LYNQHNPGASAA, EQLSNMIVSCK, or LSNMIVRSCKCS which peptide is between 4 and 18 amino acids in length.

In a second embodiment, the invention relates to a pharmaceutical composition comprising TGF-β1 and at least one synthetic peptide derived from TGF-β1 and mimicking TGF-β1 activity, which peptide comprises at least 4 consecutive amino acids from one of the sequences KNCCVRQLYIDF, NCCVRQLYIDFR, CCVRQLYIDFRK, CVRQLYIDFRKD, VRQLYIDFRKDL, RQLYIDFRKDLG, HEPKGYHANFCL, EPKGYHANFCLG, TQYSKVLALYNQ, YSKVLALYNQH, KVLALYNQHNP, NQHNPGASAAPC, QHNPGASAAPCC, HNPGASAAPCCV, PKVEQLSNMIVR or LSNMIVRSCKCS, which peptide is between 4 and 18 amino acids in length, together with at least one suitable excipient for administration.

The synthetic peptides of the present invention are derived from a TGF-β1 sequence as described in Derynk et al (Nature (1985), 701). In the present description the numbering of the amino acids of this sequence according to Derynk et al (Nature (1985), 701) is used.

Peptides derived from TGF-β1 variants, which peptides differ from the afore-mentioned peptides in amino acid sequence are within the scope of the present invention if they show the same type of activity as the peptides of the invention and are derived from a corresponding site in the variant.

Any amino acids extending on either side of the given sequences of 12 amino acids should preferably also be derived from TGF-β1 sequences either from the sequence described by Derynk et al. (Nature (1985), 701) or variants thereof which show a similar activity.

In addition, it is possible to have some conservative changes in the amino acid sequences as long as the desired effects are obtained.

In the prior art, a number of TGF-β derived sequences are described. EP-A-0 128 849 describes a partial sequence of β type TGF. In detail, the first 29 amino acids of TGF-β are given. In fact, this application is directed to TGF-β itself.

EP-A-0 511 830 relates to the sequence DFRKDIGWKW starting at position 23 of the TGF-β sequence. This sequence represents a heparin binding region, and is used in a method of separating the anticoagulant activity of heparin.

The international patent application 94/15949 is directed to two sequences, a 6 amino acid sequence depicted as GWXWXX starting at position 29, and a 8 amino acid sequence MXVXXCXC. These peptides can be used to develop products for the detection or treatment of cell proliferative disorders of the uterus or skeletal tissue. Antisense sequences are said to be useful in the treatment of uterine neoplasm, endometriosis, or skeletal disorders. Further, it is indicated that the products described can be used in contraception, in vitro fertilisation or in preventing premature labour.

The Japanese publication 6-25288 is directed to peptides starting from position 69. The peptides described are said to be useful in the treatment of fractures, wounds, multiple sclerosis, rheamatoid arthritis, metatypical arthrosis, osteoporosis and periodontitis. Further, the peptides can suppress rejective reactions after transplantations.

Finally, reference is made to WO 90/14359. Herein peptides are described derived from the region between positions 16 and 31 of the TGF-β sequence. The peptides can be used for treating immune or inflammatory disorders, e.g. allograft rejecton, septic shock, adult respiratory disease syndrome, autoimmune disease, rheumatoid arthritis, systemic lupus erythematosus or inflammatory bowel disease. In addition, the peptides can be used in wound healing and as immunogens to elicit antibodies.

Taking into account the teaching of these prior art documents, it can be concluded that there apparently is no relation between the peptide fragments shown and hence no guidance to which other parts of the TGF-β sequence may either mimick or inhibit the TGF activity.

In addition to the novel peptides and the pharmaceutical compositions of the invention as described above, the present invention also relates to a pharmaceutical composition having TGF-β1 type activity, comprising at least one of the novel peptides of the invention together with at least one suitable excipient for administration. The novel peptides of the invention can in addition be used in a method of treatment of the human or animal body, for example for repairing tissues or for immune modulation, which method comprises the application of these novel peptides.

Finally, the present invention relates to a method of treatment of the human or animal body comprising the application of TGF-β and at least one synthetic peptide, which peptide comprises at least 4 consecutive amino acids from one of the sequences KNCCVRQLYIDF, NCCVRQLYIDFR, CCVRQLYIDFRK, CVRQLYIDFRKD, VRQLYIDFRKDL, RQLYIDFRKDLG, HEPKGYHANFCL, EPKGYHANFCLG, TQYSKVLALYNQ, YSKVLALYNQH, KVLALYNQHNP, NQHNPGASAAPC, QHNPGASAAPCC, HNPGASAAPCCV, PKVEQLSNMIVR or LSNMIVRSCKCS, which peptide is between 4 and 18 amino acids in length.

The length of the peptides of the invention or used in accordance with the present invention is between 4 and 18 amino acids, preferably between 6 and 18. In the context of the present invention, this means that this number of amino acids needs to be derived from TGF-β1 or variants thereof. The peptide fragments may however be incorporated in a carrier peptide, or linked through a linker group to another TGF sequence.

If the peptides of the invention or used in the practice of the invention are derived from a shorter or longer TGF sequence the agonistic and/or synergistic activity thereof is no longer specific and/or decreases to too low a level. In other words, the activity found seems well-defined by the areas of the sequences given.

In preferred embodiments, the peptide fragments of the present invention are of a shorter length than 18 amino acid residues, e.g. peptides of 16, or preferably of 12 amino acids. These embodiments are preferred because of the well localized activity and because of ease of production.

Below the length of about 4 amino acids no specific activity is found. It is however possible to make some conservative changes to a 6 amino acid length peptide and still find specific activity as a TGF agonist, whilst not having at least 4 amino acid sequences identical to those given in the sequences above. Still such a sequence should be considered as being derived from a specified sequence according to the invention and therefore be considered as being a peptide according to the invention. This particularly goes for peptides which have two or more stretches of 3 amino acids of the sequences identified above, linked by one or more different amino acids. Thus the minimum length is quite critical, however it may not be always directly apparent that a peptide does indeed meet that criterion. For higher activity and/or specificity it is of course preferred that longer peptides of for instance 6, preferably 8, or more residues be used. Most preferred are sequences of a length of 12 amino acids, of which as many as possible are identical to the ones in the given sequences, although some conservative changes may be made, as well as some chemical modifications for, e.g., instance stability. Most preferred are the novel peptides which consist of the sequences selected from CFSSTEKNCCVR, FSSTEKNCCVRQ, EKNCCVRQLYID, NCCVRQLYIDFR, CCVRQLYIDFRK, LYIDFRKDLGWK, FRKDLGWKWIHE, RKDLGWKWIHEP, HEPKGYHANFCL, TQYSKVLALYNQ, YSKVLALYNQHN, LYNQHNPGASAA, EQLSNMIVSCK, or LSNMIVRSCKCS because these represent the center of TGF agonistic activity, or selected from KNCCVRQLYIDF, NCCVRQLYIDFR, CCVRQLYIDFRK, CVRQLYIDFRKD, VRQLYIDFRKDL, RQLYIDFRKDLG, HEPKGYHANFCL, EPKGYHANFCLG, TQYSKVLALYNQ, QYSKVLALYNQH, YSKVLALYNQHN, NQHNPGASAAPC, QHNPGASAAPCC, HNPGASAAPCCV, PKVEQLSNMIVR or LSNMIVRSCKCS, because these represent the centers of the synergistic activity if combined with TGF-β.

The peptides of the invention or used according to the invention may include modified amino acids on the termini (or in the sequence itself for that matter) in order to increase the resistance to breakdown, or otherwise enhance the stability and/or the half-life. The peptides according to the invention may also be circularized for the same or for another purpose.

If the peptides of the invention are incorporated into longer structures, it is preferred that they not be incorporated at the termini of the structures in order to avoid unwanted degradation.

The novel peptides of the present invention and the peptides used in the pharmaceutical compositions of the present invention can be prepared using methods which are known per se, e.g. solid phase synthesis, fragment condensation or recombinant DNA techniques. Suitable prior art methods are for instance described by Steward and Young, Solid Phase Synthesis, Pierce Chemical Company, Rockfort, Illinois (1984), and by Valerio et al. in Int. J. Peptide Protein Res. 42 (1993) 1-9, and in Int. J. Peptide Protein Res. 44 (1994) 158-165.
The novel peptides can be used as TGF agonists. This has been illustrated in in vitro bio-assays as will be shown in the examples described herein below. More in detail, the peptides having the sequences CFSSTEKNCCVR, FSSTEKNCCVRQ, EKNCCVRQLYID, NCCVRQLYIDFR, CCVRQLYIDFRK, LYIDFRKDLGWK, FRKDLGWKWIHE, RKDLGWKWIHEP, HEPKGYHANFCL, TQYSKALALYNQ, YSKVLALYNQHN, LYNQHNPGASAA, EQLSNMIVSCK, or LSNMIVRSCKCS inhibited the growth of mink lung epithelial cells in a similar way as TGF-β1 does. In addition, it is illustrated that peptides having one of the sequences KNCCVRQLYIDF, NCCVRQLYIDFR, CCVRQLYIDFRK, CVRQLYIDFRKD, VRQLYIDFRKDL, RQLYIDFRKDLG, HEPKGYHANFCL, EPKGYHANFCLG, TQYSKVLALYNQ, YSKVLALYNQH, KVLALYNQHNP, NQHNPGASAAPC, QHNPGASAAPCC, HNPGASAAPCCV, PKVEQLSNMIVR or LSNMIVRSCKCS give rise to an enhanced inhibition of the growth of the mink lung epithelial cells when combined with a sub optimal dose of TGF-β1, which shows that these peptide sequences show a synergistic effect, when used in combination with TGF-β1.

In the pharmaceutical compositions of the present invention at least one of the peptides of the invention is present together with a suitable excipient, and optionally TGF-β. Usually the peptides will be given by injection (parenteral administration), or enterally, but most likely not orally since oral formulations of peptides are quite difficult to prepare. Orally the peptides can basically only be given if they are protected from proteolytic breakdown in the stomach. This is feasible, but until now not very practical. Upon parenteral administration the composition must be in liquid form, e.g. in the form of a real liquid or of a suspension or emulsion. The formulation of the peptide may however be prepared as a dry preparation to be reconstituted with a suitable liquid. The dry preparations as well as liquid formulations may comprise several additives for increasing ease of administration, storage capability, resistence to breakdown, enhancing bio-availability, increasing half-life, providing isotonic preparations, etc. Usual additives for the preparation of pharmaceutical compositions, whether they be dry or liquid are well known in the art. Many peptides have been formulated in many kinds of preparations. For instance LHRH peptides and analogues have been formulated for many purposes and in many variations. In principle most of the formulations presenting peptides as drugs (not in vaccines) will be useful, depending on the kind, amount and duration of the TGF agonistic action desired. Doses to be given depend on a number of parameters, not all of which can be given before clinical trials have been done. The skilled artisan however knows how to arrive at suitable doses for different treatments. Usually dose finding is first done in animal studies using escalating doses to determine efficacy and acute toxicity. Thereafter long term effects of the drugs are studied after which clinical trials in the three wellknown phases follow. It is expected that the doses to be given eventually will lie between 0.01 and 10 mg/kg, preferably between 1 and 10 mg/kg, bodyweight per day, preferably between 7 and 7000 mg per day, most preferably between 70 and 700 mg per day.

The invention will now be illustrated in the following examples.

### EXAMPLES

### Introduction

### 1. Peptide synthesis:

The peptides used in the following examples where synthesized following in the method described in the afore mentioned articles of Valerio et al.

More in detail, peptides are synthesized onto polyethylene pins (or surfaces) applying standard Merrifield synthesis. All overlapping 12 amino acid long peptides were produced covering the length of the TGF-β1 amino acid sequence. After synthesis, the peptides are split off the pins (or surfaces) using trifluoroacetic acid (TFA) and freed from contaminants by ether extractions and freeze drying.

### 2. Assay for agonistic TGF activity.

An assay for TGF-β1 biological activity, bases on Mink lung epithelial cells (Mv 1 Lu), was used for analysis of the biological activity of peptides derived from the human TGF-β1 primary sequence. This assay is based on growth inhibition of Mv 1 Lu cells in presence of TGF-β1. Inhihition of Mv 1 Lu growth is quantified by acid phosphatase activity as a marker for cell growth.

Mink lung epithelial cells (Mv 1 Lu) were plated in 96-well tissue culture plates (Costar, Cambridge, MA) at 100 cells/well in 100 µl Eagle's MEM supplemented with 20IE/ml penicillin, 1 mg/ml streptomycin, 0.4 mM L-glutamine, 10 mM HEPES and 10% FCS (HyClone, Logan, UT). Plates were incubated at 37°C and 5% CO₂ in a humidified incubator for 1.5 h to allow cell attachment. After cell attachment peptides were added in a volume of 50 µl in Eagle's MEM supplemented with 20IE/ml penicillin, 1 mg/ml streptomycin, 0.4 mM L-glutamine, 10 mM HEPES and 10% FCS and titrated with eight threefold dilutions of peptide. The cells were subsequently incubated for 5 days at 37°C and 5% CO₂ in a humidified incubator. After incubation cell growth was monitored using acid phosphatase activity as a marker: The plates were washed twice with 200 µl PBS per well and 100 µl of substrate solution (0.1 M sodium acetate, 0,1% Triton X-100, 3.7 mg/ml p-nitrophenyl phosphate, pH 5.5) were added to each well. The plates were then incubated for 1.5 h at 37°C, after which 25 µl of 0.4 M NaOH were added to each well. Acid phosphatase activity was detected by measuring the OD of the substrate solution using an microplate reader at 405 nm.

### Example 1

In this example, all peptides comprising 12 amino acids prepared as described under the head "Peptide synthesis", were tested in the Mv 1 Lu growth inhibition assay. In this assay peptides were titrated in eight threefold dilutions, starting with a peptide quantity of 10-100 µg per well. After 5 days of incubation the wells were tested for acid phosphatase activity.

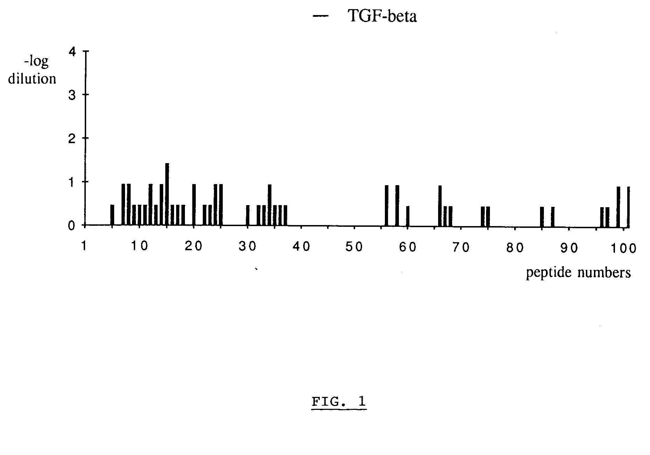

As shown in Figure 1 a number of unique agonistic peptides, mimicking TGF-β1 activity, were identified. Furthermore, previously published agonistic peptides were also identified in this experiment. No peptides with antagonistic activity were identified.

The observed growth inhibition of Mv 1 Lu cells induced by the identified peptides is still detectable in this experiment at a peptide concentration of 6.1 µM when 10 µg of peptide is present as starting material before dilution.

### Example 2

In this example Mv 1 Lu cells were preincubated with TGF-β1 derived peptides which were titrated in eight threefold dilutions, starting with a peptide quantity of 10-100 µg per well. After 4 h of preincubation a suboptimal quantity of 3.3 x 10⁻³ µM TGF-β1. The TGF-β1 (R&D systems, M1) Porcine TGF-β1 is 100% homologue to humam TGF-β1 (Roberts et al. in Handbook of Experimental Pharmacology, Editors Sporn, M.B.; Robers A.B.; Springer Verlag Heidelberg (1990), 95, 419) was added. After 5 days of incubation the wells were tested for acid phosphatase activity.

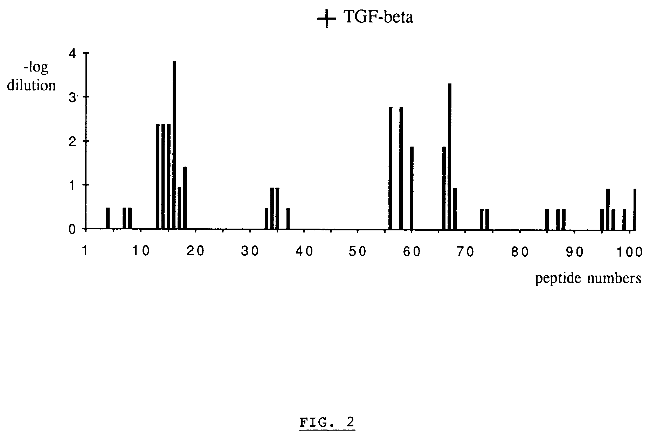

Figure 2 shows the result of this experiment. Although this experiment was designed to identify TGF-β1 antagonistic peptides only agonistic peptides were identified, in mostly similar regions of the TGF-β1 molecule as the unique agonistic peptides, identified in Example 1. Furthermore similar, previously agonistic peptides, similar to those identified in Example 1 were also identified and extended in this experiment.

The observed growth inhibition of Mv 1 Lu cells induced by the identified peptides is still detectable in this experiment at a peptide concentration of 2.3*10⁻¹ µM - 8*10⁻² µM when 10 µg of peptide is present as starting material before dilution. Interestingly, preincubation with growth inhibiting peptides to Mv 1 Lu cells, followed by addition of 3.3*10⁻³ µM TGF-β1, results in a synergistic effect upon growth inhibition of Mv 1 Lu cells when compared to growth inhibition when peptides or TGF-β1 (3.3*10⁻³ µM) only are added to Mv 1 Lu cells (compare Example 1).

The best peptides display at maximal growth inhibition an activity which is about 10³ less than TGF-β1 activity on a molar basis.

Interestingly, the peptides display, with TGF-β1, a synergistic effect on the inhibition of Mv 1 Lu cell growth. This suggest that unique combinations of peptides from various regions in the TGF-β1 molecule may be active simultaneously.

### Legend to the Figures:

In Figure 1 the agonistic activity of overlapping peptides derived from TGF-β1 is shown;
In Figure 2, the agonistic activity of overlapping peptides derived from TGF-β1 is shown in the presence of a suboptimal dose of TGF-β1.

## Claims

1. A synthetic peptide derived from TGF-β and mimicking TGF-β activity, which peptide comprises at least 4 consecutive amino acids from one of the sequences CFSSTEKNCCVR, FSSTEKNCCVRQ, EKNCCVRQLYID, NCCVRQLYIDFR, CCVRQLYIDFRK, LYIDFRKDLGWK, FRKDLGWKWIHE, RKDLGWKWIHEP, HEPKGYHANFCL, TQYSKVLALYNQ, YSKVLALYNQHN, LYNQHNPGASAA, EQLSNMIVSCK, or LSNMIVRSCKCS and which peptide is between 4 and 18 amino acids in length.

2. The peptide of claim 1 having a length of between 6 and 18 amino acids.

3. The peptide of claim 1 or 2, having a sequence selected from CFSSTEKNCCVR, FSSTEKNCCVRQ, EKNCCVRQLYID, NCCVRQLYIDFR, CCVRQLYIDFRK, LYIDFRKDLGWK, FRKDLGWKWIHE, RKDLGWKWIHEP, HEPKGYHANFCL, TQYSKVLALYNQ, YSKVLALYNQHN, LYNQHNPGASAA.EQLSNMIVSCK, or LSNMIVRSCKCS

4. A pharmaceutical composition having TGF-β type activity, comprising a peptide of any one of the preceding claims together with at least one suitable excipient for administration.

5. A pharmaceutical composition comprising TGF-β1 and at least one synthetic peptide derived from TGF-β and mimicking TGF-β activity, which peptide comprises at least 4 consecutive amino acids from one of the sequences KNCCVRQLYIDF, NCCVRQLYIDFR, CCVRQLYIDFRK, CVRQLYIDFRKD, VRQLYIDFRKDL, RQLYIDFRKDLG, HEPKGYHANFCL, EPKGYHANFCLG, TQYSKVLALYNQ, YSKVLALYNQH, KVLALYNQHNP, NQHNPGASAAPC, QHNPGASAAPCC, HNPGASAAPCCV, PKVEQLSNMIVR or LSNMIVRSCKCS, which peptide is between 4 and 18 amino acids in length, together with at least one suitable excipient for administration.

6. The composition of claim 5, wherein at least one of said synthetic peptides has a length of 6-18 amino acids.

7. The composition of claim 5 or 6, wherein at least one of said synthetic peptides is selected from KNCCVRQLYIDF, NCCVRQLYIDFR, CCVRQLYIDFRK, CVRQLYIDFRKD, VRQLYIDFRKDL, RQLYIDFRKDLG, HEPKGYHANFCL, EPKGYHANFCLG, TQYSKVLALYNQ, YSKVLALYNQH, KVLALYNQHNP, NQHNPGASAAPC, QHNPGASAAPCC, HNPGASAAPCCV, PKVEQLSNMIVR or LSNMIVRSCKCS.

8. A method of treatment of the human or animal body comprising the application of TGF-β and at least one synthetic peptide, which peptide comprises at least 4 consecutive amino acids from one of the sequences KNCCVRQLYIDF, NCCVRQLYIDFR, CCVRQLYIDFRK, CVRQLYIDFRKD, VRQLYIDFRKDL, RQLYIDFRKDLG, HEPKGYHANFCL, EPKGYHANFCLG, TQYSKVLALYNQ, YSKVLALYNQH, KVLALYNQHNP, NQHNPGASAAPC, QHNPGASAAPCC, HNPGASAAPCCV, PKVEQLSNMIVR or LSNMIVRSCKCS, which peptide is between 4 and 18 amino acids in length.


SEQUENCE LISTING

(1) GENERAL INFORMATION:

(i) APPLICANT:
(A) NAME: Instituut voor Dierhouderij en Diergezondheid
(ID-DLO)
(B) STREET: Edelhertweg 15
(C) CITY: Lelystad
(D) STATE: Flevoland
(E) COUNTRY: the Netherlands
(F) POSTAL CODE (ZIP): 8219 PH

(ii) TITLE OF INVENTION: TGF-B1 derived peptides mimicking the
activity of Transforming Growth Factor-B1.

(iii) NUMBER OF SEQUENCES: 28

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97201342.9

(2) INFORMATION FOR SEQ ID NO: 1:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 1:

Cys Phe Ser Ser Thr Glu Lys Asn Cys Cys Val Arg
1               5                   10

(2) INFORMATION FOR SEQ ID NO: 2:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 2:

Phe Ser Ser Thr Glu Lys Asn Cys Cys Val Arg Gln
1               5                   10

(2) INFORMATION FOR SEQ ID NO: 3:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 3:

Glu Lys Asn Cys Cys Val Arg Gln Leu Tyr Ile Asp
1               5                   10

(2) INFORMATION FOR SEQ ID NO: 4:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 4:

Asn Cys Cys Val Arg Gln Leu Tyr Ile Asp Phe Arg
1               5                   10

2) INFORMATION FOR SEQ ID NO: 5:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 5:

Cys Cys Val Arg Gln Leu Tyr Ile Asp Phe Arg Lys
1               5                   10

(2) INFORMATION FOR SEQ ID NO: 6:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 6:

Leu Tyr Ile Asp Phe Arg Lys Asp Leu Gly Trp Lys
1               5                   10

(2) INFORMATION FOR SEQ ID NO: 7:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 7:

Phe Arg Lys Asp Leu Gly Trp Lys Trp Ile His Glu
1               5                   10

2) INFORMATION FOR SEQ ID NO: 8:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 8:

Arg Lys Asp Leu Gly Trp Lys Trp Ile His Glu Pro
1               5                   10

(2) INFORMATION FOR SEQ ID NO: 9:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 9:

His Glu Pro Lys Gly Tyr His Ala Asn Phe Cys Leu
1               5                   10

(2) INFORMATION FOR SEQ ID NO: 10:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 10:

Thr Gln Tyr Ser Lys Val Leu Ala Leu Tyr Asn Gln
1               5                   10

2) INFORMATION FOR SEQ ID NO: 11:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 11:

Tyr Ser Lys Val Leu Ala Leu Tyr Asn Gln His Asn
1               5                   10

(2) INFORMATION FOR SEQ ID NO: 12:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 12:

Leu Tyr Asn Gln His Asn Pro Gly Ala Ser Ala Ala
1               5                   10

(2) INFORMATION FOR SEQ ID NO: 13:

(i) SEQUENCE CHARACTERISTICS:
(A) LENGTH: 11 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 13:

Glu Gln Leu Ser Asn Met Ile Val Ser Cys Lys
1               5                   10

2) INFORMATION FOR SEQ ID NO: 14:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 14:

Leu Ser Asn Met Ile Val Arg Ser Cys Lys Cys Ser
1               5                   10

(2) INFORMATION FOR SEQ ID NO: 15:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 15:

Lys Asn Cys Cys Val Arg Gln Leu Tyr Ile Asp Phe
1               5                   10

(2) INFORMATION FOR SEQ ID NO: 16:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 16:

Cys Val Arg Gln Leu Tyr Ile Asp Phe Arg Lys Asp
1               5                   10

2) INFORMATION FOR SEQ ID NO: 17:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 17:

Val Arg Gln Leu Tyr Ile Asp Phe Arg Lys Asp Leu
1               5                   10

(2) INFORMATION FOR SEQ ID NO: 18:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 18:

Arg Gln Leu Tyr Ile Asp Phe Arg Lys Asp Leu Gly
1               5                   10

2) INFORMATION FOR SEQ ID NO: 19:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 19:

Glu Pro Lys Gly Tyr His Ala Asn Phe Cys Leu Gly
1               5                   10

2) INFORMATION FOR SEQ ID NO: 20:

(i) SEQUENCE CHARACTERISTICS:
(A) LENGTH: 11 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 20:

Tyr Ser Lys Val Leu Ala Leu Tyr Asn Gln His
1               5                   10

(2) INFORMATION FOR SEQ ID NO: 21:

(i) SEQUENCE CHARACTERISTICS:
(A) LENGTH: 11 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 21:

Lys Val Leu Ala Leu Tyr Asn Gln His Asn Pro
1               5                   10

(2) INFORMATION FOR SEQ ID NO: 22:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 22:

Asn Gln His Asn Pro Gly Ala Ser Ala Ala Pro Cys
1               5                   10

2) INFORMATION FOR SEQ ID NO: 23:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 23:

Gln His Asn Pro Gly Ala Ser Ala Ala Pro Cys Cys
1               5                   10

(2) INFORMATION FOR SEQ ID NO: 24:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 24:

His Asn Pro Gly Ala Ser Ala Ala Pro Cys Cys Val
1               5                   10

(2) INFORMATION FOR SEQ ID NO: 25:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 25:

Pro Lys Val Glu Gln Leu Ser Asn Met Ile Val Arg
1               5                   10

2) INFORMATION FOR SEQ ID NO: 26:

(i) SEQUENCE CHARACTERISTICS:
(A) LENGTH: 10 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 26:

Asp Phe Arg Lys Asp Ile Gly Trp Lys Trp
1               5                   10

(2) INFORMATION FOR SEQ ID NO: 27:

(i) SEQUENCE CHARACTERISTICS:
(A) LENGTH: 6 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(ix) FEATURE:
(A) NAME/KEY: Peptide
(B) LOCATION:3..6
(D) OTHER INFORMATION:/product= "X"
/note= "X represents any amino acid."

(xi) SEQUENCE DESCRIPTION: SEQ ID NO: 27:

Gly Trp Xaa Trp Xaa Xaa
1               5

2) INFORMATION FOR SEQ ID NO: 28:

(i) SEQUENCE CHARACTERISTICS:
(A) LENGTH: 8 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(ix) FEATURE:
(A) NAME/KEY: Peptide
(B) LOCATION:2..7
(D) OTHER INFORMATION:/product= "X"
/note= "X represents any amino acid."

(xi) SEQUENCE DESCRIPTION: SEQ ID NO: 28:

Met Xaa Val Xaa Xaa Cys Xaa Cys
1               5